# EUROPÄISCHE PATENTSCHRIFT

(11) **EP 3 243 532 B1**
(45) Veröffentlichungstag und Bekanntmachung des Hinweises auf die Patenterteilung: **25.11.2020**
(21) Anmeldenummer: 17170372.1
(22) Anmeldetag: 10.05.2017
(51) Int. Cl.: D01F 8/12, A61L 9/014, D01F 8/14, A61L 9/02, A61L 9/03, D01D 5/34

(54) **DUFTSTOFFKÖRPER**
FRAGRANCE BODY
CORPS ODORANT

(30) Priorität: 13.05.2016 DE 202016102602 U
(43) Veröffentlichungstag der Anmeldung: 15.11.2017
(73) Patentinhaber: Mantz GmbH, 42651 Solingen (DE)
(72) Erfinder: Seefeld, Jörg, 42659 Solingen (DE)
(74) Vertreter: Kudla, Christof

(56) Entgegenhaltungen:
- WO-A1-2007/136795
- WO-A1-2011/123723
- WO-A1-2013/025585
- US-A1- 2003 211 799

## Beschreibung

Die vorliegende Erfindung betrifft einen Duftstoffkörper.

Zur Lufterfrischung werden heute Duftmittel vielfältig insbesondere im Haushalt oder beispielsweise auch in Kraftfahrzeugen verwendet. Herkömmliche Duftstoffkörper können beispielsweise durch Mischen eines Riechstoffs (Parfüm) mit einem Kunststoffgranulat (Beladen) und nachfolgendem Spritzgießen des jeweiligen Duftstoffkörpers hergestellt werden. Der Duftstoff ist so homogen in dem Duftstoffkörper verteilt. Darüber hinaus ist insbesondere für Haushaltszwecke bekannt, zur Lufterfrischung Duftlampen zu verwenden, welche eine, beispielsweise durch ein Teelicht erhitzte Duftschale aufweisen, in die ätherische Öle bzw. Duftstoffe oder gelöste Duftstoffe im flüssigen Aggregatzustand eingebracht werden, wobei durch die Duftstoffe durch Verdunstung in den gasförmigen Zustand übergehen und somit die gewünschte Lufterfrischung erzeugen.

Während die angegebenen Duftstoffkörper, bei welchen der Duftstoff in einen Kunststoffkörper eingebunden ist, nur eine sehr reduzierte Duftabgabe bereitstellen kann, besteht bei der Verwendung von Duftlampen häufig das Problem, dass die Flüssigkeit bei unsachgemäßer Handhabung der Duftlampe verschüttet und insofern beispielsweise Teppiche oder Möbel verschmutzt werden können.

WO 2011/123723 A1, WO 2007/136795 A1, US 2003/211799 A1, WO 2013/025585 A1 offenbaren Duftstoffträgerkörper mit einem bimodalen Faseraufbau mit Monokomponentenfasern und/oder Zweikomponentenfasern (Kern/Mantel).

Der vorliegenden Erfindung liegt die Aufgabe zugrunde, die beschriebenen Nachteile bei der Durchführung einer Lufterfrischung zumindest teilweise zu beheben. Die Erfindung löst diese Aufgabe mit einem Duftstoffkörper, umfassend einen Trägerkörper, welcher eine Stoff- oder Flüssigkeitsmischung, umfassend zumindest ein Lösungsmittel und zumindest ein in diesem gelöstes Parfum (Riechstoff) im Volumen trägt, wobei der Duftstoffkörper zur Abgabe von Duft bei Erwärmung ausgebildet ist und der Trägerkörper eine Faserstrukturierung mit einem bimodalen Faseraufbau aufweist, und wobei eine erste Faserart einen Polyamidkern mit einer Polyesterumhüllung umfasst und eine zweite Faserart eine nicht ummantelte Polyamid-Faser umfasst.

Der erfindungsgemäße Duftstoffkörper kann durch das Vorsehen einer Faserstrukturierung, umfassend im Wesentlichen zwei unterschiedliche Faserarten, einen Träger für die Stoff- oder Flüssigkeitsmischung, umfassend zumindest ein Lösungsmittel und zumindest ein in diesem gelöstes Parfum bereitstellen, in welchem die Mischung im Volumen gebunden ist und bei Zimmertemperatur sowie bei erhöhten Temperaturen bis etwa 150°C oder gar 200°C nicht flüssig aus dem Trägerkörper austritt. Ein Verschütten der das Parfum enthaltenden Stoff- oder Flüssigkeitsmischung ist somit ausgeschlossen. Darüber hinaus kann durch das Vorsehen einer erhöhten Temperatur des Duftstoffkörpers die Duftabgabe durch Einstellen der Temperatur in einer Duftlampe, beispielsweise durch Vorsehen einer bestimmten Beabstandung der Wärmequelle, die durch ein Teelicht bereitgestellt sein kann, zum Duftstoffkörper, festgelegt werden. Dabei hat sich zur Einstellung einer vorteilhaften Faserstrukturierung des Trägerkörpers das Vorsehen eines bimodalen Faseraufbaus als erfindungsgemäß herausgestellt. Durch die Verwendung einer ersten Faserart, umfassend einen Polyamidkern mit einer Polyesterumhüllung und einer zweiten Faserart, umfassend eine Polyamidfaser kann die Stoff- oder Flüssigkeitsmischung optimal im Volumen des Trägerkörpers gehalten werden. Ferner weisen die verwendeten Fasern die notwendige Temperaturresistenz auf, sodass beispielsweise ein Verbrennen oder Verkokeln des Trägerkörpers bei der Verwendung des erfindungsgemäßen Duftstoffkörpers sicher ausgeschlossen werden kann.

Weiterbildungen der Erfindung und weitere erfindungsgemäße Merkmale sind in der allgemeinen Beschreibung, der Figurenbeschreibung sowie in den Zeichnungen angegeben.

Um die Rückhaltung der den Riechstoff beinhaltenden Stoff- oder Flüssigkeitsmischung im Trägerkörper weiter zu verbessern, kann vorgesehen sein, dass die Faserstrukturierung des Trägerkörpers eine Kapillarstruktur durch eine entsprechende Anordnung der zwei Faserarten aufweist.

Eine zweckmäßige Faserstrukturierung kann dadurch erzielt werden, dass sich die Fasern beider Faserarten im Wesentlichen um eine vorbestimmte Richtung, insbesondere hin und her oder in Zickzackart erstrecken, wobei die Faserlänge etwa ±15% der Erstreckung des Trägerkörpers in die vorbestimmte Richtung betragen kann. Durch diese Maßnahme kann die Gestaltung einer Vielzahl von Kapillaren im Trägerkörper erreicht werden, die sich im Wesentlichen in eine Richtung, beispielsweise in Richtung der Höhe des Trägerkörpers erstrecken.

Es ist erfindungsgemäß ganz besonders bevorzugt, wenn die Faserlänge 10 bis 20% der Erstreckung des Trägerkörpers in die vorbestimmte Richtung beträgt.

Es hat sich als besonders zweckmäßig für die Tragfähigkeit und die Duftabgabe bei erhöhter Temperatur herausgestellt, wenn der Tragkörper etwa 70% Gewichtsanteil Polyamid und etwa 30% Gewichtsanteil Polyester aufweist.

Es hat sich als ganz besonders zweckmäßig für die Tragfähigkeit und die Duftabgabe bei erhöhter Temperatur herausgestellt, wenn der Tragkörper 60 bis 80 Gew-% Polyamid und 40 bis 20 Gew.-% Polyester aufweist, bevorzugt wenn der Tragkörper 65 bis 75 Gew-% Polyamid und 35 bis 25 Gew.-% Polyester aufweist, bezogen auf das Gesamtgewicht des Tragkörpers.

Zweckmäßigerweise kann der erfindungsgemäße Duftstoffkörper zur Erzielung eines bestimmten optischen Eindruckes eingefärbt sein.

Die Gestalt des Trägerkörpers kann an die jeweilige Verwendung, beispielsweise für die Verwendung in einer Duftlampe angepasst sein. Besonders zweckmäßig hat sich ein zylinderförmig gestalteter Trägerkörper herausgestellt, wobei dessen Abmessungen an die jeweilige Anwendung angepasst sein können. Dabei kann beispielsweise der Durchmesser des zylinderförmigen Trägerkörpers bis zu 30 mm betragen, insbesondere bei 19,5 mm oder 25,5 mm liegen. In gleicher Weise kann die Höhe des Trägerkörpers angepasst sein und beispielsweise zwischen 3 mm und 5 mm liegen.

Besonders zweckmäßig kann es sein, wenn der Trägerkörper aus einer zylinderförmigen Urform ausgestanzt bzw. ausgeformt ist, oder in einer speziellen Form extrudiert wird, sodass der Trägerkörper in Bezug auf seine Grundfläche eine beliebige Strukturierung und an die jeweilige Anwendung angepasste Form aufweisen kann. Insbesondere bei der Extrusion des Trägerkörpers kann dieser eine beliebige Grundfläche aufweisen, die den Querschnitt senkrecht zur Extrusionsrichtung des Trägerkörpers festlegt.

Um ein bestimmtes Hohlraumvolumen zur Aufnahme der den Riechstoff aufweisenden Stoff- bzw. Flüssigkeitsmischung bereitzustellen, kann der Trägerkörper des erfindungsgemäßen Duftstoffkörpers eine Dichte aufweisen, die zwischen 0,14 g/cm³ bis 0,19 g/cm³ liegt, beispielsweise 0,15 g/cm³ oder 0,18 g/cm³ betragen.

Es hat sich als zweckmäßig herausgestellt, wenn die Faserdicke der unbeschichteten Polyamidfaser einen Wert aufweist, der zwischen 8 µm und 12 µm liegt, insbesondere 9 µm beträgt oder zwischen 10 µm und 12 µm liegt.

Um die Funktionsfähigkeit des erfindungsgemäßen Duftstoffkörpers auch bei erhöhten Temperaturen im Bereich von etwa 80 bis 130° oder gar 150°C bereitzustellen, kann zweckmäßigerweise vorgesehen sein, dass alle Bestandteile des Trägerkörpers bei einer Temperatur von 220°C im festen Aggregatzustand befinden.

Bei der Herstellung des erfindungsgemäßen Duftstoffkörpers kann zweckmäßigerweise vorgesehen sein, dass der Trägerkörper mit der den Duftstoff enthaltenden Stoff- oder Flüssigkeitsmischung getränkt ist. Diese Tränkung kann zweckmäßigerweise bei einer zur Zimmertemperatur erhöhten Temperatur von z.B. 80 °C erfolgen, die zweckmäßigerweise unterhalb der Siedetemperatur des Duftmittels liegen kann. Diese kann beispielsweise zwischen 90°C und 100°C liegen, insbesondere etwa 95°C betragen.

Vorzugsweise kann dabei vorgesehen sein, dass das zumindest eine Lösungsmittel eine Siedetemperatur > 220°, insbesondere > 230° aufweist.

Die Erfindung betrifft ferner einen verpackten Duftstoffkörper, bei dem ein erfindungsgemäßer Duftstoffkörper luftdicht verpackt, insbesondere in einem luftdichten Sachet angeordnet ist. Die Erfindung betrifft darüber hinaus eine Duftvorrichtung, insbesondere eine Duftlampe mit einer Duftschale, in welche ein erfindungsgemäßer Duftstoffkörper einsetzbar ist.

Die Erfindung wird im Folgenden durch das Beschreiben einiger Ausführungsformen unter Bezugnahme auf die beiliegenden Zeichnungen erläutert, wobei
- Figuren 1a - c: unterschiedliche geometrische Gestaltungen eines erfindungsgemäßen Duftstoffkörpers in einer perspektivischen Ansicht, einer Aufsicht sowie einer Seitenansicht,
- Figur 2a, b: den Einschluss eines erfindungsgemäßen Duftkörpers in einem Sachet in einer geschlossenen Form zum Transport sowie geöffnet und
- Figur 3: die Verwendung eines erfindungsgemäßen Duftstoffkörpers in einer beispielhaften Duftlampe zeigt.

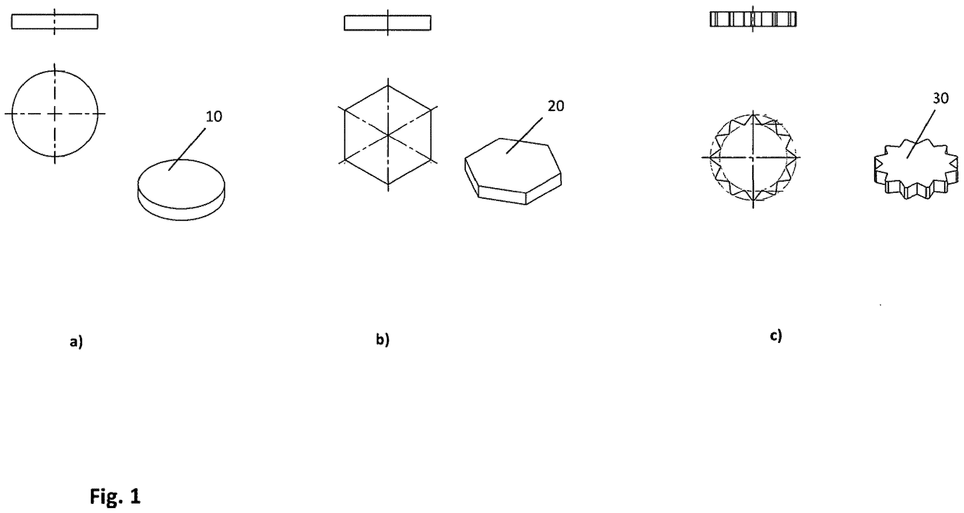

Figuren 1a - c zeigen drei unterschiedlich geformte erfindungsgemäße Duftstoffkörper 10, 20, 30, jeweils in einer perspektivischen Ansicht, einer Aufsicht sowie einer Seitenansicht. Diese unterscheiden sich allein in Bezug auf ihre jeweilige Grundfläche. Letztlich kann der erfindungsgemäße Duftstoffkörper mit einer beliebigen Grundfläche ausgebildet sein. Darüber hinaus ist es auch möglich, den Duftstoffkörper in Bezug auf die dritte Dimension, d.h. seine Höhe komplex auszubilden, sodass der Duftstoffkörper grundsätzlich eine beliebige Gestalt einnehmen kann.

All diesen Varianten ist gemein, dass ein jeweiliger Trägerkörper, welcher die geometrische Gestalt des Duftstoffkörpers festlegt, mit einer Stoff- oder Flüssigkeitsmischung getränkt ist, wobei diese Mischung zumindest ein Lösungsmittel und zumindest ein in diesem gelöstes Parfum, d.h. ein Riechstoff umfasst.

Erfindungsgemäß ist der Trägerkörper als Faserstrukturierung mit einem bimodalen Faseraufbau gestaltet, wobei die erste Faserart eine mit Polyester umhüllte Polyamidfaser und die zweite Faserart eine nicht ummantelte Polyamidfaser darstellt. Die Fasern der beiden Faserarten sind in der beschriebenen Ausführungsform so zueinander angeordnet, dass sich eine Kapillarstruktur über das gesamte Volumen des Trägerkörpers ergibt, in welcher die beschriebene Stoff- oder Flüssigkeitsmischung aufgenommen ist. Dabei erstrecken sich alle Fasern im Trägerkörper im Wesentlichen um eine vorbestimmte Richtung, d.h. hin und her um diese Richtung, wobei die jeweilige Faserlänge etwa ± 15% (von 10 bis 20%, vorzugsweise von 12 bis 18%) der Erstreckung des Trägerkörpers in die vorbestimmte Richtung beträgt. In der beschriebenen Ausführungsform ist diese vorbestimmte Richtung parallel zur Höhenrichtung der Duftstoffkörper orientiert, sodass diese Richtung auch die Richtung der durch die Faserstrukturierung gebildeten Kapillare festlegt.

In den beschriebenen Ausführungsformen weist der Trägerkörper in einer ersten Variante eine Dichte von 0,15 g/cm³ und in einer zweiten Variante eine Dichte von 0,18g/cm³ auf. Die beiden Varianten unterscheiden sich insofern in Bezug auf die Kapillargrößen und können zur Optimierung der Tragfähigkeit des Trägerkörpers in Bezug auf die physikalischen Eigenschaften der Stoff- oder Flüssigkeitsmischung ausgewählt werden. In der beschriebenen Ausführungsform weisen die im Wesentlichen zylindrischen Fasern eine Dicke zwischen 10 und 12 µm auf.

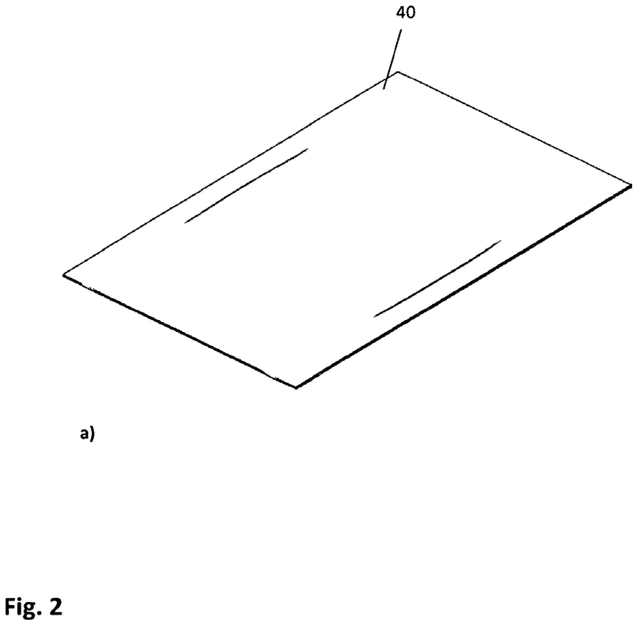

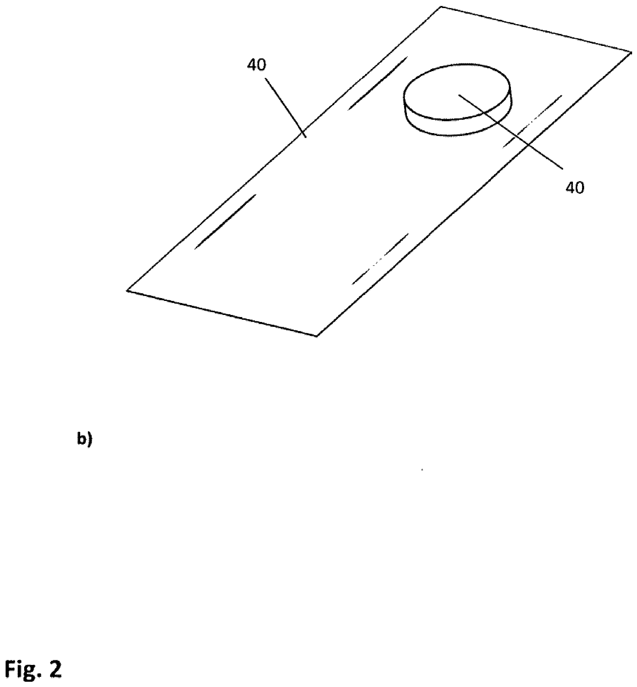

Figuren 2a, b zeigen das Verpacken eines erfindungsgemäßen Duftstoffkörpers 10 in einem luftdicht verschlossenen Sachet 40 (Figur 2a), wobei Figur 2b das Sachet in einem geöffneten Zustand mit dem Duftkörper 10 zeigt. In der beschriebenen Ausführungsform ist das Sachet aus einer Kunststofffolie gestaltet.

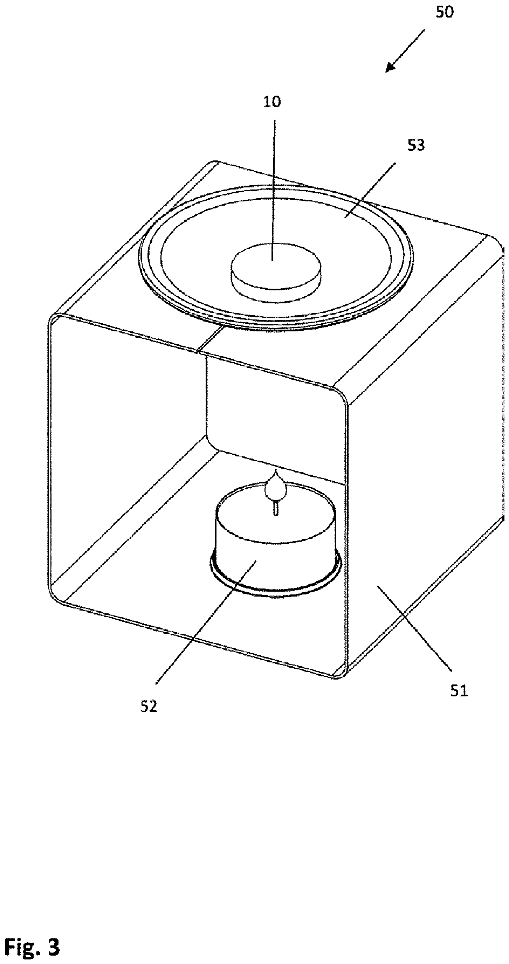

Figur 3 zeigt die beispielhafte Verwendung eines erfindungsgemäßen Duftstoffkörpers 10 in einer Duftlampe 50. Die Duftlampe 50 umfasst ein kubusförmiges Gestell 51, das zu zwei Seiten offen gestaltet ist und an seinem inneren Boden ein Teelicht 52 trägt, das eine an der Oberseite des Traggestells platzierte Duftschale 53 erhitzt, in welche ein erfindungsgemäßer Duftstoffkörper 10 eingesetzt ist.

Durch die Erwärmung des Duftstoffkörpers 10 und des darin getragenen Gemischs erhöht sich die Verdunstungsrate des Duftstoffes, wobei die physikalischen Eigenschaften des Stoff- oder Flüssigkeitsgemischs und des Trägerkörpers so eingestellt sind, dass bei der durch das Teelicht erzeugten Betriebstemperatur das Gemisch im Trägermaterial verbleibt, sodass die Duftschale 53 trocken bleibt und keine Gefahr besteht, dass bei der Handhabung der Duftlampe Flüssigkeit verschüttet wird.

In einer nicht dargestellten Ausführungsform kann die Duftlampe auch elektrisch betrieben werden, beispielsweise mittels einer elektrischen Heizung, welche die Duftschale erwärmt oder durch ein elektrisch betriebenes, beispielsweise als Glühlampe ausgebildetes Leuchtmittel, das seine Abwärme über Wärmestrahlung an die Duftschale bzw. den Duftstoffkörper abgibt.

### Bezuaszeichenliste

- 10,20,30: Duftstoffkörper
- 40: Sachet
- 50: Duftlampe
- 51: Gestell
- 52: Teelicht
- 53: Duftschale

## Patentansprüche

1. Duftstoffkörper (10, 20, 30) umfassend einen Trägerkörper, welcher eine Stoff- oder Flüssigkeitsmischung umfassend zumindest ein Lösungsmittel und zumindest ein in diesem gelöstes Parfüm im Volumen trägt, wobei der Duftstoffkörper zur Abgabe von Duft bei Erwärmung ausgebildet ist und der Trägerkörper eine Faserstrukturierung mit einem bimodalen Faseraufbau aufweist, **dadurch gekennzeichnet, dass** eine erste Faserart einen Polyamidkern mit einer Polyesterumhüllung umfasst und eine zweite Faserart eine nicht ummantelte Polyamid-Faser umfasst.

2. Duftstoffkörper (10, 20, 30) nach Anspruch 1, **dadurch gekennzeichnet, dass** die Faserstrukturierung des Trägerkörpers eine Kapillarstruktur umfasst.

3. Duftstoffkörper (10, 20, 30) nach Anspruch 1 oder 2, **dadurch gekennzeichnet, dass** der Trägerkörper 60 bis 80 Gew-% Polyamid und 40 bis 20 Gew.-% Polyester aufweist, bezogen auf das Gesamtgewicht des Trägerkörpers.

4. Duftstoffkörper (10, 20, 30) nach einem der Ansprüche 1 bis 3, **dadurch gekennzeichnet, dass** der Trägerkörper zylinderförmig aufgebaut ist oder eine mehreckige Grundfläche aufweist.

5. Duftstoffkörper (10, 20, 30) nach einem der Ansprüche 1 bis 4, **dadurch gekennzeichnet, dass** der Trägerkörper aus einer zylinderförmigen Urform ausgestanzt bzw. ausgeformt oder der Trägerkörper extrudiert ist.

6. Duftstoffkörper (10, 20, 30) nach einem der Ansprüche 1 bis 5, **dadurch gekennzeichnet, dass** der Trägerkörper eine Dichte zwischen 0,14 g/cm³ bis 0,19 g/cm³ aufweist.

7. Duftstoffkörper (10, 20, 30) nach einem der Ansprüche 1 bis 6, **dadurch gekennzeichnet, dass** die Faserdicke der unbeschichteten Polyamidfaser einen Wert aufweist, der zwischen 8 µm und 12 µm, insbesondere bei 9 µm oder zwischen 10 µm und 12 µm liegt.

8. Duftstoffkörper (10, 20, 30) nach einem der Ansprüche 1 bis 7, **dadurch gekennzeichnet, dass** alle Bestandteile des Trägerkörpers bei einer Temperatur von 220°C im festen Aggregatzustand vorliegen.

9. Duftstoffkörper (10, 20, 30) nach einem der Ansprüche 1 bis 8, **dadurch gekennzeichnet, dass** der Trägerkörper mit der Stoff- oder Flüssigkeitsmischung umfassend zumindest ein Lösungsmittel und zumindest ein in diesem gelösten Parfüm getränkt ist.

10. Duftstoffkörper (10, 20, 30) nach einem der Ansprüche 1 bis 9, **dadurch gekennzeichnet, dass** das zumindest eine Lösungsmittel eine Siedetemperatur größer 220°C aufweist.

11. Verpackter Duftstoffkörper, bei dem ein Duftstoffkörper nach einem der Ansprüche 1 bis 10 in einer luftdichten Verpackung angeordnet ist.

12. Duftstoffeinrichtung, insbesondere Duftlampe (50), mit einer beheizbaren Duftschale (53), sowie einem Duftstoffkörper (10, 20, 30) nach einem der Ansprüche 1 bis 10 zum Einsetzen in die Duftschale.

## Claims

1. Fragrance body (10, 20, 30) comprising a carrier body, which carries in the volume a mixture of a substance or a liquid comprising at least one solvent and at least one perfume dissolved in the latter, the fragrance body being designed for dispensing fragrance when heated and the carrier body having a fibre structuring with a bimodal fibre structure, **characterized in that** a first type of fibre comprises a polyamide core with a polyester coating and a second type of fibre comprises an uncoated polyamide fibre.

2. Fragrance body (10, 20, 30) according to Claim 1, **characterized in that** the fibre structuring of the carrier body comprises a capillary structure.

3. Fragrance body (10, 20, 30) according to Claim 1 or 2, **characterized in that** the carrier body comprises 60 to 80% by weight polyamide and 40 to 20% by weight polyester, with respect to the total weight of the carrier body.

4. Fragrance body (10, 20, 30) according to one of Claims 1 to 3, **characterized in that** the carrier body is constructed in a cylindrical form or has a polygonal base area.

5. Fragrance body (10, 20, 30) according to one of Claims 1 to 4, **characterized in that** the carrier body is punched out or moulded from a cylindrical original form or the carrier body is extruded.

6. Fragrance body (10, 20, 30) according to one of Claims 1 to 5, **characterized in that** the carrier body has a density of between 0.14 g/cm³ and 0.19 g/cm³.

7. Fragrance body (10, 20, 30) according to one of Claims 1 to 6, **characterized in that** the fibre thickness of the uncoated polyamide fibre has a value that lies between 8 µm and 12 µm, in particular at 9 µm or between 10 µm and 12 µm.

8. Fragrance body (10, 20, 30) according to one of Claims 1 to 7, **characterized in that** all of the constituent parts of the carrier body are in the solid state of aggregation at a temperature of 220°C.

9. Fragrance body (10, 20, 30) according to one of Claims 1 to 8, **characterized in that** the carrier body is impregnated with the mixture of a substance or a liquid comprising at least one solvent and at least one perfume dissolved in the latter.

10. Fragrance body (10, 20, 30) according to one of Claims 1 to 9, **characterized in that** the at least one solvent has a boiling temperature greater than 220°C.

11. Packaged fragrance body, in which a fragrance body according to one of Claims 1 to 10 is arranged in an airtight packaging.

12. Fragrance device, in particular a fragrance lamp (50), with a heatable fragrance shell (53), and also a fragrance body (10, 20, 30) according to one of Claims 1 to 10 for fitting into the fragrance shell.

## Revendications

1. Corps odorant (10, 20, 30) comprenant un corps porteur qui porte dans le volume un mélange de substances ou de liquides comprenant au moins un solvant et au moins un parfum dissous dans celui-ci, le corps odorant étant conçu pour libérer un parfum lorsqu'il est chauffé et le corps porteur présentant une structuration fibreuse pourvue d'une structure fibreuse bimodale,
**caractérisé en ce que**
un premier type de fibre comprend une âme en polyamide pourvue d'une gaine en polyester et un deuxième type de fibre comprend une fibre de polyamide non gainée.

2. Corps odorant (10, 20, 30) selon la revendication 1, **caractérisé en ce que** la structuration fibreuse du corps porteur comprend une structure capillaire.

3. Corps odorant (10, 20, 30) selon la revendication 1 ou 2, **caractérisé en ce que** le corps porteur comprend 60 à 80 % en poids de polyamide et 40 à 20 % en poids de polyester, par rapport au poids total du corps porteur.

4. Corps odorant (10, 20, 30) selon l'une des revendications 1 à 3, **caractérisé en ce que** le corps porteur est réalisé sous la forme d'un cylindre ou comporte une base polygonale.

5. Corps odorant (10, 20, 30) selon l'une des revendications 1 à 4, **caractérisé en ce que** le corps porteur est estampé ou conformé à partir d'une forme originale cylindrique ou le corps porteur est extrudé.

6. Corps odorant (10, 20, 30) selon l'une des revendications 1 à 5, **caractérisé en ce que** le corps porteur a une densité comprise entre 0,14 g/cm³ et 0,19 g/cm³.

7. Corps odorant (10, 20, 30) selon l'une des revendications 1 à 6, **caractérisé en ce que** l'épaisseur de la fibre polyamide non gainée a une valeur qui est comprise entre 8 µm et 12 µm, en particulier de 9 µm ou entre 10 µm et 12 µm.

8. Corps odorant (10, 20, 30) selon l'une des revendications 1 à 7, **caractérisé en ce que** tous les constituants du corps porteur sont présents à l'état solide à une température de 220 °C.

9. Corps odorant (10, 20, 30) selon l'une des revendications 1 à 8, **caractérisé en ce que** le corps porteur est imprégné du mélange de substances ou liquides comprenant au moins un solvant et au moins un parfum dissous dans celui-ci.

10. Corps odorant (10, 20, 30) selon l'une des revendications 1 à 9, **caractérisé en ce que** l'au moins un solvant a une température d'ébullition supérieure à 220 °C.

11. Corps odorant emballé, un corps odorant selon l'une des revendications 1 à 10 étant disposé dans un emballage hermétique.

12. Dispositif odorant, en particulier lampe de parfum (50), comprenant un bol de parfum (53) pouvant être chauffé et un corps odorant (10, 20, 30) selon l'une des revendications 1 à 10 destiné à être inséré dans le bol de parfum.
